# EUROPEAN PATENT APPLICATION

(11) **EP 4 844 372 A1**
(43) Date of publication of application: **25.11.2026**
(21) Application number: 25382501.2
(22) Date of filing: 19.05.2025
(51) Int. Cl.: G01N 21/03, G01N 21/552, G01N 21/05, B01L 3/00, G01N 33/543

(54) **A PLASMONIC SENSOR CARTRIDGE, A POINT-OF-CARE DETECTION SYSTEM COMPRISING SAID CARTRIDGE, AND A METHOD FOR QUANTITATIVE ANALYTE IDENTIFICATION USING SAID SYSTEM**

(71) Applicant: Fundació Institut Català de Nanociència i Nanotecnologia (ICN2), 08193 Cerdanyola del Vallès (Barcelona) (ES); Consejo Superior de Investigaciones Científicas (CSIC), 28006 Madrid (ES)
(72) Inventor: SOLER AZNAR, Maria, E-08310 Argentona (Barcelona) (ES); LECHUGA GÓMEZ, Laura, E-08173 San Cugat del Vallès (Barcelona) (ES); ESTÉVEZ ALBEROLA, Mª Carmen, E-08025 Barcelona (ES)
(74) Representative: ABG Intellectual Property Law, S.L.

(57) **Abstract**

The invention is directed to a plasmonic sensor cartridge and a point-of-care detection system for quantitative analyte identification in a sample using said plasmonic sensor cartridge. The cartridge comprises a sample reservoir, a microfluidic cuvette, and a plasmonic sensor with functionalized regions for selective analyte binding. The sensor features a nanopatterned substrate with defined pit depth and separation, coated with a metal layer supporting surface plasmon resonance. The invention also refers to a method for quantitative analyte identification which is carried out by said system.

## Description

### FIELD OF THE INVENTION

This invention lies within the scope of a point-of-care system using a multiplexed plasmonic (bio)sensor capable of real-time monitoring and analysis of multiple biomolecular interactions within the same sample. The invention presents a simple, economical solution using readily available and low-cost optical components for ultrasensitive biological and chemical sensing. Such a system could be used for academic and industrial research, industrial testing, and crucially in clinical laboratories and point-of-care healthcare testing to improve diagnostics and treatment decisions.

### BACKGROUND

In the prior art, Thirstrup et al. ("Diffractive optical coupling element for surface plasmon resonance sensors", DOI: 10.1016/j.snb.2004.01.010, published in Sensors and Actuators B: Chemical in 2004) disclose an early implementation of diffractive optical coupling elements integrated onto the plasmonic biosensor itself for multiplexed sensing. This approach utilizes prism-coupler-based surface plasmon resonance (SPR) sensing and necessitates a specially structured SPR chip, which can introduce increased manufacturing complexity and potentially higher production costs.

Vala et al. ("High-performance compact SPR sensor for multi-analyte sensing", DOI: 10.1016/j.snb.2010.05.067, published in Sensors and Actuators B: Chemical in 2010) also describe a microfluidic, multichannel sensing device designed for multiplexed SPR measurements. This device employs different illumination configurations and optical fibres, which can lead to increased system complexity and potential issues related to optical alignment and stability.

Consequently, the known alternatives often suffer from drawbacks such as significant bulk, the requirement of moving or dynamically adjusted components, or the integration of intricate optical fibre networks (as exemplified by Vala et al.).

Therefore, a significant need remains in the state of the art to provide a portable, rapid, highly sensitive, and label-free testing method capable of analyzing analytes in parallel from a single, low-volume sample. Such a solution would be highly advantageous for a wide array of applications, including early disease diagnostics, prompt infection alerts, biological sample screening, explosive detection, water quality testing, and gas sensing, among others.

### DESCRIPTION OF THE INVENTION

The invention is based on plasmonic sensing, which allows such rapid, sensing and label-free testing. At the same time, the invention overcomes limitations of existing implementations that suffer from poor multiplexed ability, complicated light coupling mechanisms and restrictive size implementation with bulky optics.

In this way, the present invention provides a plasmonic sensor cartridge according to claim 1, a point-of-care (POC) detection system for quantitative analyte identification in a sample according to claim 9, and a method for quantitative analyte identification according to claim 15. Preferred embodiments of the invention are set out in dependent claims.

In a first inventive aspect, the invention provides *a plasmonic sensor cartridge for a point-of-care detection system for quantitative analyte identification in a sample to be analyzed, the cartridge comprising:*
- *a sample reservoir adapted to contain the sample to be analyzed;*
- *a plasmonic sensor configured to detect the concentration* of a *specific analyte of the sample, the plasmonic sensor comprising at least one functionalized region;*
- *a microfluidic cuvette in fluid communication with the plasmonic sensor and configured to distribute the sample from the sample reservoir towards the at least one functionalized region;*
*wherein the plasmonic sensor comprises:*
- *a nanopatterned substrate comprising a plurality of pits separated by lands; wherein the pit depth is in the range* of 25 *to 50 nm and the separation between adjacent pits is in the range of 200 to 750 nm;*
- *a metal surface coating the nanopatterned substrate and having a thickness between 50-150 nm, the metal surface being configured to support surface plasmon resonance, wherein the metal surface is functionalized for the selective binding of the analyte.*

In the scope of this invention, functionalization would be construed as the process of modifying the sensor's surface with specific molecules to enable it to selectively bind to a target molecule. For instance, a gold surface coating arranged onto the nanostructured substrate of a Blu-ray disc-based plasmonic sensor can be modified or functionalized to detect a specific analyte (such as a protein). Only the target analyte will bind to the functionalized surface (also referred to throughout the description as "active regions") of the plasmonic sensor, while other molecules in the sample will not (or will bind minimally). This specificity is essential for biosensing applications, where the goal is to detect the presence and concentration of a particular analyte in a sample.

In the invention, the metallic surface is functionalized with a bioreceptor capable of selectively binding to an analyte. For instance, the bioreceptor may be an antibody.

Multispot illumination, in optics, refers to a technique where a single light source is used to create an array of multiple, discrete light spots on a target surface. This is achieved using optical elements that manipulate the light beam. In a preferred embodiment, a diffractive optical element (DOE) is used to obtain this multispot illumination. The DOE splits the incoming light into several beams, each of which forms a spot at a specific location on the target (in this case, the plasmonic sensor's surface).

The cartridge according to the first inventive aspect can be either consumable or reusable. In an embodiment, the cartridge is embodied as a fungible element and is replaced for each sample test.

In this way, the invention provides a compact, versatile cartridge for a point-of-care detection system featuring a plasmonic biosensor, engineered for multiplexed detection of human and biological analytes.

Unlike traditional plasmonic-based analyte detection systems that rely on prism coupling, the plasmonic sensor cartridge achieves light in- and out-coupling through a plasmonic nanostructure on the surface of the biosensor. This simple nanostructured coupling simplifies the design and reduces costs and moveable parts.

For instance, the specific analyte of the sample may be organic molecules, proteins, oligonucleotides or combinations thereof.

In an embodiment, the nanopatterned substrate is a 1D, 2D or 3D array of pits.

In an embodiment, the microfluidic cuvette comprises a single microfluidic sample channel with a microfluidic input and a microfluidic output adapted to transfer the sample across the at least one functionalized region.

In an embodiment, the microfluidic cuvette comprises multiple microfluidic sample channels on a same cartridge (3), said multiple microfluidic sample channels putting in fluid communication a plurality of microfluidic inputs and corresponding microfluidic outputs.

In an embodiment, the plasmonic sensor comprises an optional nanometric adhesion layer arranged between the nanopatterned substrate and the metal surface coating. In a preferred embodiment, the thickness of the nanometric adhesion layer is of a few nanometers, preferably between 1 and 5 nm. The nanometric adhesion layer may be made of Ti, Cr, etc.

In an embodiment, the plasmonic sensor is fabricated by depositing a plasmonic metal material, such as Au, Ag, or Al (or combinations thereof), onto a substrate (for example, glass or polymer), utilizing a nanometric adhesion layer (for instance, composed of Ti or Cr) in between.

In an embodiment, the microfluidic cuvette is manufactured from a biocompatible polymer, for example: polydimethylsiloxane (PDMS), SU-8 (a commonly used epoxy-based negative photoresist), polymethylmethacrylate (PMMA), cyclic olefin copolymer (COC), and polycarbonate (PC). In preferred embodiments, the microfluidic cuvette comprises one of the following combinations of materials: PMMA/PDMS, PMMA/COC, PMMA/SU8/PDMS, PC/COP, PC/COC. However, this list is not exhaustive, and other biocompatible polymers, along with combinations of various materials, may also be employed.

In an embodiment, the metal surface is made of Au, Ag, Al or combinations of these materials.

In this invention, the design of the plasmonic sensor nanostructure is related to the illumination source parameters, such as the incidence light angle and wavelength.

Based on light diffraction on a high ordered periodically nanostructured surface, the light wave is split into reflected and diffracted waves. The high-order periodic structures like the ones present in Blu-rays discs and other optical discs behave like diffraction gratings allowing the possibility to use them as couplers to generate plasmonic effect when they are coated with a thin metallic layer. This principle is applied in the cartridge and system according to the present invention.

For the generation of plasmons in a dielectric substrate coated with a thin metallic film (in this case, the nanopatterned substrate coated with the metal surface), the light is diffracted parallel to the interface by the grating allowing the matching between the wavevector of the diffracted light and that of the plasmonic effect in the thin metallic layer. The plasmonic effect induced by the grating coupling can be described by the Bloch wave surface plasmon resonance (BW-SPR), when the Bragg condition for one-dimensional periodic metallic structure is satisfied. Under oblique-angle incident light, it can be described by the following equation: ${λ}_{SPR} \left(n, i, θ\right) = \frac{P}{i} \left\{Re\left[{\left(\frac{{ε}_{m} {n}^{2}}{{ε}_{m} + {n}^{2}}\right)}^{1 / 2}\right]\pm sinθ\right\}$

Plasmonic band can be tuned by varying the following parameters: P, which is the period of the structure (in this case, the structure is the nanopatterned substrate coated with the surface coating); εₘ, which is the relative permittivity of the metal surface coating material; and/or θ, which is the illumination incidence angle. In the previous equation, "i" is the resonant order. For the embodiments of the invention discussed later, it is assumed i=1.

In the equation, the dielectric constant of gold (εₘ= -10 +i1). If we neglect the imaginary part, at a fixed grating period of for example 320 nm, for n=1.33 (water) and normal angle (sinθ=0) the wavelength is approximately 460 nm. This effect will increase at higher angles of incidence (sinθ>0). Likewise, the displacement will also increase at longer wavelengths (red shift), if the angle is kept fixed and the refractive index n increases. Thus, from the previous expression, a red shift in the resonant wavelength is expected when the refractive index (denoted as n) of the medium near the gold surface increases and with longer nanostructure periods. Also, for a fixed nanostructure period, the resonance wavelength can be either controlled by the metal surface coating employed (for instance, by varying their thickness and material) or by modifying the light incidence angle.

The equation above details the relationship between the angle and wavelength of the incident illumination and the material properties of the nanostructure. For instance, a person skilled in the art can determine the most suitable combinations of these parameters, such as wavelength, through 3D Finite-difference time-domain (FDTD) simulations and by referring to the disclosure of this patent application. The examples provided herein typically utilize a laser with a wavelength around 660 nm. However, the invention is not limited to this specific wavelength and can be implemented using other lasers operating in the visible or near-infrared spectrum. It's important to note that modifications to the illumination source (e.g., changing the wavelength) may necessitate slight adjustments to the nanostructure design or the angle of incidence.

In an embodiment, the microfluidic cuvette comprises one or more microfluidic channels integrated with the metal surface.

In alternative embodiments of the plasmonic sensor cartridge, the microfluidic cuvette is in fluid communication with external microfluidics. In these embodiments, the point-of-care detection system comprising the cartridge has the rest of microfluidic elements (pumps, reservoirs, etc.) out of the cartridge, for instance, in a microfluidic distribution unit.

In an embodiment, the microfluidic channel is formed in a PDMS layer and defined by a double-sided adhesive tape.

In an embodiment, *the plasmonic sensor cartridge further comprises a casing integrating the sample reservoir, the microfluidic cuvette and the plasmonic sensor.*

Advantageously, integrating all the components into a single casing makes the cartridge easier to handle, insert into the point-of-care system, and dispose of. This simplifies the user workflow and reduces the risk of mishandling individual components of the cartridge.

In an embodiment, *the metal surface is made* of a *plasmonic metal, the plasmonic metal preferably comprising gold, silver or aluminium; and the metal surface has* a *thickness between 50 and 150 nm.*

In preferred embodiments, the plasmonic metal of the metal surface comprises gold, silver or aluminium. Still, this list of plasmonic metals is not exhaustive.

In an embodiment, *the nanopatterned substrate comprises one of the following: a Blu-ray disc, polymeric nanopatterned substrate, a metallic nanograting, a silica nanograting.*

In a preferred of the cartridge, the plasmonic sensor is obtained by a manufacturing process comprising the steps of:
- providing a commercial Blu-ray disc comprising a nanostructured polycarbonate substrate;
- removing the upper reflective and protective layer of the Blu-ray disc; and
- directly vaporizing a metal surface coating over the nanostructured polycarbonate substrate of the Blu-ray disc.

In a preferred embodiment, the nanopatterned substrate of the plasmonic sensor is a Blu-ray disc, leveraging its nanostructured profile as an alternative manufacturing approach. Advantageously, using Blu-ray discs as substrates allows to reduce cost and provide a scalable production, making this invention compact, economical, and suitable for diverse applications in diagnostics and industrial testing.

In an embodiment, *the microfluidic cuvette comprises: a plurality of microfluidic channels to route the sample from the reservoir towards the at least one functionalized region, and a waste reservoir.* In this embodiment, the at least one waste reservoir is configured to receive the sample (and optionally, additional reagents or buffer solutions applied to said sample) after it has interacted with the plasmonic sensor. Advantageously, a dedicated waste reservoir prevents the analyzed sample from flowing back into the sample reservoir or interfering with subsequent measurements.

In an embodiment, the plasmonic sensor cartridge comprises an integrated waste reservoir. Preferably, the volume of the waste reservoir is larger than the volume of the sample reservoir. More preferably, the volume of the waste reservoir is between 100-400 µL.

In alternative embodiments, the waste reservoir is external to the plasmonic sensor cartridge. In some embodiments, the waste reservoir is placed in a microfluidic distribution unit.

In an embodiment, *the plasmonic sensor cartridge is manufactured using one or more biocompatible materials selected from the group consisting of polydimethylsiloxane (PDMS), SU-8 (an epoxy-based photoresist commonly used in microfabrication), polymethylmethacrylate (PMMA), cyclic olefin copolymer or cyclic olefin polymer* (COC/COP), *polycarbonate (PC), polytetrafluoroethylene (PTFE), perfluoroalkoxy alkane (PFA), and fluorinated ethylene propylene (FEP).* The use of these specific biocompatible materials ensures compatibility with biological samples and/or environments, contributing to the reliable operation and application of the plasmonic sensor cartridge.

In an embodiment, *the plasmonic sensor comprises a plurality of functionalized regions functionalized for the selective binding of different analytes in the sample.*

In an embodiment of the plasmonic sensor cartridge:
- *the cartridge is sealed to prevent leakage of the sample; and*/*or*
- *the volume of the sample reservoir is between 50-150 µL.*

The plasmonic sensor cartridge of the first inventive aspect is configured to provide direct optical coupling between the plasmonic sensor and a monochromatic illumination source.

In a second inventive aspect, the invention refers to *a point-of-care detection system for quantitative analyte identification in a sample, the system comprising:*
*a) the plasmonic sensor cartridge according to any one of the first inventive aspect embodiments;*
*b) a measurement device comprising one or more of the following:*
   - *a microfluidic distribution unit configured to route the sample to the plasmonic sensor cartridge; wherein the microfluidic cuvette of the cartridge is in fluid communication with said microfluidic distribution unit;*
   - *a monochromatic illumination source configured to emit light within the visible to near infrared spectrum range and to couple light into the at least one functionalized region of the plasmonic sensor;*
   - *optical arrangement configured to polarize the light from the illumination source in TM mode and to direct the polarized light onto at least onto the metal surface of the plasmonic sensor at an oblique angle of incidence;*
   - *a detection module optically coupled with the plasmonic sensor cartridge and configured to measure the intensity of the light reflected from at least one functionalized region of the metal surface of the plasmonic sensor after interaction of the polarized light with said plasmonic sensor;*
*c) a processing unit communicably coupled to the measurement device and configured to:*
   - *convert the reflected light intensity into a voltage signal; and*
   - *provide a quantitative output representative of the concentration of the analyte based on said voltage signal.*

In an embodiment of the point-of-care detection system, the optical arrangement is configured to induce a plasmonic resonance peak in the plasmonic sensor. Particularly, the optical arrangement is configured to polarize the illumination in transverse-magnetic (TM). Surface plasmons are excited by light that has its magnetic field component transverse (perpendicular) to the direction of propagation and parallel to the plane of incidence. Thus, TM-polarized light is required to drive the oscillation of electrons at the metal surface needed for surface plasmon excitation.

Surface plasmon polaritons (SPPs) propagate at the interface between a metal and dielectric (in this case, the substrate) and are only excited by the TM mode of incident light. This is due to the physical principle of plasmonic operation. Thus, to induce the plasmonic resonance peak, it is required that the plasmonic sensor is excited by a TM mode of the incident light. More preferably, the resonance is induced at an angle between 20 and 85 degrees. This angle refers to the angle in which light impinges onto the metal surface of the plasmonic sensor.

The key principle of the point-of-care detection system is that the binding of analyte molecules to the functionalized sensor surface causes a change in the optical properties (i.e. the refractive index) of the plasmonic sensor, and this change is then measured and correlated to the analyte concentration.

The metallic layer of the sensor is functionalized with a bioreceptor (e.g., an antibody) that is specific to the analyte of interest. This means the bioreceptor will selectively bind to the analyte molecules. When a sample containing the analyte is introduced to the sensor, the analyte molecules bind to the bioreceptors on the sensor surface. This binding event causes a change in the refractive index in the vicinity of the plasmonic sensor surface. When molecules bind to the surface, they alter the local optical refractive index. The change in refractive index affects the surface plasmon resonance (SPR) condition. Specifically, it causes a shift in the resonance peak of the reflected light.

The detection module measures the reflected light, and the processing unit determines the changes in the spectral characteristics of this reflected light. By analyzing the shift in the resonance peak, the concentration of the analyte can be determined.

In short, the system of the invention is able to measure analyte concentration by detecting changes in the reflected light that are caused by the analyte binding to the plasmonic sensor surface.

In some embodiments, the measurement device of the system is a reusable element, whereas in some embodiments the cartridge is conceived as a fungible element. The measurement device comprises the illumination source, the optical arrangement and the optional active microfluidic distribution unit, if present. The measurement device is the part of the system which controls and performs the measurement on the plasmonic sensor cartridge after the introduction of the sample. The measurement device may comprise the illumination source, detectors, optics, active elements, processing, connectivity and graphical interface.

The point-of-care detection system employs a crucial modular design where the plasmonic sensor cartridge is conceived as a fungible (disposable) element, intended for single use with a sample. This eliminates the risk of cross-contamination between tests and simplifies handling for the user. Conversely, the measurement device, housing the core analytical components like the illumination source, optics, detectors, and processing unit, is designed as a reusable element. This separation allows for cost-effective operation, as only the low-cost cartridge needs to be replaced for each test. This modular approach, with a disposable cartridge interfacing with a reusable reader, is essential for a practical and scalable point-of-care diagnostic solution, balancing cost-efficiency with stringent hygiene requirements and ease of use. Still, in other embodiments of the invention, the plasmonic sensor cartridge can be reusable too.

In an embodiment, the system further comprises a structural support designed to securely clamp the plasmonic sensor cartridge and the microfluidic distribution unit. This support may be also configured to provide mechanical movement for adjusting the spatial position of the plasmonic sensor cartridge. In preferred embodiments, the structural support is operably connected to a high-precision rotation stage, enabling a complete 360° rotation of the plasmonic sensor cartridge. An embodiment of this rotation stage includes a micrometer with a minimum resolution of 5 arcminutes. This capability advantageously permits fine-tuning of the cartridge's rotational orientation within the system, thereby facilitating the identification of the optimal incidence angle for the illumination source interacting with the plasmonic sensor to induce the surface plasmon resonance.

In an embodiment of the system, the optical arrangement is configured to induce a plasmonic resonance peak in the metal surface of the plasmonic sensor at an oblique illumination angle between 20 and 85 degrees.

In an embodiment, the measurement device comprises an active microfluidic distribution unit configured to provide controlled continuous sample flow and to enable automated handling of aqueous samples if a plasmonic sensor cartridge requiring active flow control is used. The active microfluidic distribution unit may comprise:
- Pumps: Sample flow towards the plasmonic sensor cartridge can be provided with various types of pumps, such as peristaltic, syringe, or capillary pumps, which provide a stable, continuous flow during the analysis.
- Injection valve: This valve allows for precise injection of test samples into the flow loop, enabling sequential introduction of samples and reagents as needed.
- Reservoirs: After passing over the plasmonic sensor, the sample flows into a biocompatible waste reservoir (either on cartridge or external and in fluid communication with the measurement device) for safe disposal. Optional on-cartridge or off-cartridge reservoirs contain buffer, reagents or other solutions to aid the measurement process.

Preferably, this microfluidic distribution unit allows for the handling of very small sample volumes (down to 50 microliters) and could support processes like sample washing, regeneration of the biorecognition layer, and real-time sample introduction, enhancing the system's adaptability to different diagnostic requirements.

Advantageously, the system has a sensitivity of approximately 10⁻⁵ refractive index units (RIUs), and enables rapid, cost-effective analysis, making it ideal for point-of-care diagnostics and for versatile applications in clinical diagnostics, academic research, or industrial settings.

In an embodiment of the system, the monochromatic illumination source within the visible to near infrared spectrum range emits light with wavelength between 550-850 nm, and said illumination source is configured to illuminate directly and to couple light into the at least one functionalized region in a reflection-based scheme.

In a preferred embodiment of the system, the plasmonic sensor cartridge and the measurement device comprise complementary alignment features for precisely positioning the plasmonic sensor cartridge within the measurement device, whereby the complementary alignment features are selected from the group comprising: grooves and ridges, pins and holes, keyways and slots, or magnetic couplings.

In an embodiment of the system, the oblique angle of incidence is in the range 20 to 85 degrees.

In an embodiment, *the monochromatic illumination source of the system is a laser diode configured to emit at a wavelength in the visible and*/*or near-infrared range around 660 nm.*

In the context of the present invention, the visible to near-infrared (VNIR) spectrum range typically refers to the portion of the electromagnetic spectrum with wavelengths spanning approximately 340 nm to 1050 nm.

In an embodiment, *the optical arrangement further comprises:*
- *a collimator optically coupled to the monochromatic illumination source and configured to collimate a light beam emitted by the monochromatic illumination source;*
- *a polarizer optically coupled to the collimator and configured to generate a transverse magnetically polarized collimated light beam;*
- *a diffractive optical element optically coupled to the polarizer and configured to shape the polarized light beam and direct said polarized light beam towards the plasmonic sensor of the plasmonic sensor cartridge; wherein said diffractive optical element is further configured to generate a multispot illumination pattern incident on the at least one functionalized region, thereby defining a plurality of sensing spots onto the at least one functionalized region.*

Advantageously, the introduction of DOE-based multiplexing avoids dynamic parts or complex optical fibres, contrary to conventional approaches (e.g., Vala et al.). In other words, this embodiment features a novel illumination approach that uses a holographic diffractive optical element (DOE) to create multiple focused light spots on the plasmonic biosensor (embedded in cartridge), enabling simultaneous measurements of multiple analytes without the need for mechanical movement.

In this embodiment of the system, the polarizer is configured to direct polarized light towards the plasmonic sensor.

In an embodiment of the system, the optical diffraction element (DOE) is configured to split the incoming light beam into multiple, equally spaced, divergent beams with a variable number, such as 1:5, 1:11, or a greater 1:M ratio. In general, M is an integer larger than 1, which defines a multispot illumination wherein an incoming light beam is split in M equally spaced and divergent beams. In this way, the DOE operates as a multispot beam splitter. The value of M depends on how many different active regions of the plasmonic sensor need to be analyzed. This approach provides the possibility of a multiplexed analyte detection. The output of the DOE is a plurality of M sensing spots, and eventually each spot is focused onto a different functionalized region of the plasmonic sensor's surface, thereby allowing a multiplexed detection.

In the scope of the present invention, a sensing spot refers to a defined portion of the multisport illumination pattern generated by the diffractive optical element. Within each sensing spot, the interaction between the incident light and a functionalized region of the plasmonic sensor's surface is monitored to detect the binding of the target analyte. Advantageously, by having multiple sensing spots, the invention may allow for multiplexing, wherein different sensing spots illuminate different functionalized regions to detect multiple analytes simultaneously. Also, redundant measurements can be provided for a same analyte detection, for improved accuracy.

In a preferred embodiment of the system, the diffractive optical element is holographic.

In one embodiment, the optical arrangement includes an additional lens positioned in the optical path between the DOE and the plasmonic sensor. This lens is configured to correct beam divergence and maintain the spatial separation of the illumination beams from the DOE.

In an embodiment of the system, *the detection module comprises:*
- *a plurality of detection elements optically coupled with the plasmonic sensor, said detection elements being selected from the group comprising a photodiode array,* a *CMOS camera or a* CCD *camera configured to detect light in the visible to near infrared spectrum range;*
- *wherein the plurality of detection elements is configured to measure the intensity of the light reflected by the at least one functionalized region.*

In an embodiment of the system, the processing unit is configured to measure the light intensity of the sensing spots in a continuous way. In this way, the corresponding variation of the voltage signal over time allows to monitor a variation of the analyte in the sample.

In an embodiment, *the detection elements are provided* as *a photodiode array comprising up to N=16 channels, each channel being associated with a different sensing spot of the multispot illumination incident on a corresponding functionalized region of the plasmonic sensor.* This configuration, utilizing multiple sensing spots, offers the advantage of monitoring different areas on the sensor surface. This capability can be exploited to assess the analyte concentration across the sensor or, alternatively, to simultaneously detect multiple analytes if different functionalized regions are designed for specific targets.

In an embodiment, the detection elements (e.g. photodiode array) are configured to detect incident light intensity between 340 and 1050 nm.

In an embodiment, the diffractive optical element is further configured to output a plurality of collimated and transverse magnetically polarized light beams of substantially uniform intensity. Advantageously, by using mostly uniform intensity, a more consistent excitation of surface plasmons is provided. Uniform intensity across all sensing spots ensures that the surface plasmons are excited with a consistent energy level. This leads to a more homogeneous and predictable plasmon resonance phenomenon across the entire sensing area (i.e. functionalized region(s)).

In an embodiment, the detection module comprises a photodetector array with 16 channels, each channel being configured to capture reflected light from each illuminated spot on the plasmonic sensor. The photodetector array can be varied in size to match the number of illumination regions excited by the DOE. In this embodiment, this photodetector array is optimized to detect the intensity of light across the visible to near-infrared spectrum (340 - 1050 nm), with special sensitivity at the plasmonics resonance peak. Additional filters and gratings can be included in the detection module to improve resolution and maximize sensitivity at this resonance peak wavelength. Preferably, the photodetector array is fixed at an optimal angle for tracking the plasmonic resonance of the plasmonic sensor.

In other embodiments, the photodiode array may have a different number N of channels, even larger than N=16.

In an embodiment, the system further comprises: an angular displacement component configured to modify the incidence angle of the monochromatic illumination source in the plasmonic sensor and to detect an angle for tracking the plasmonic resonance of the plasmonic sensor; and an actuator to place the detection elements of the detection module based on said angle.

In an embodiment, the electronics unit is configured to get continuous and successive detection measurements in the sensing spots.

In an embodiment, *the microfluidic distribution unit is configured to deliver, in a controlled manner, the sample to the microfluidic cuvette; the microfluidic distribution unit (6) comprising:*
- *A pump configured to deliver liquid solutions at a constant flow rate to the plasmonic sensor via the microfluidic cuvette;*
- *A liquid tubing system arranged between the pump and the plasmonic sensor and configured to provide fluid communication between the pump and the plasmonic sensor;*
- *an optional injection valve in fluid communication with the liquid tubing system and configured to load and inject successive samples towards the plasmonic sensor; and*
- *a waste reservoir adapted to collect waste solutions.*

In this embodiment, the pump is configured to deliver fluid solutions (including the sample and reagents to treat said sample) via the liquid tubing system to the plasmonic sensor.

In an embodiment, the constant flow rate is between 10-100 µL/min, preferably between 20-30 µL/min.

In certain embodiments, the microfluidic distribution unit is active and includes a pump configured to inject a constant flow of saline buffer. This pump is connected to an injection valve, which is integrated into a sample loading loop.

In an embodiment, the system further comprises one or more of the following elements:
- a communication module configured to perform a data transfer with an external device;
- control electronics configured to manage the activation and intensity of the illumination source;
- a display, preferably a touchscreen, configured to display a user interface;
- a battery configured to feed the illumination source and/or the processing unit; and/or
- data storage means configured to quantitative outputs of the processing unit.

In an embodiment, the system further comprises a microfluidic distribution unit (either active or passive) configured to provide automated sample handling and supporting multiple volumes, as well as ensuring stable distribution of the sample to the plasmonic biosensor.

In an embodiment, the processing unit comprises a control and analysis software to automatically optimize the integration time, detection of spectra dip, polynomial fitting, and to provide interrogation in real-time of the reflected intensity changes in the sensing spots as output voltage collected by the detection elements.

In a third inventive aspect, the invention refers to *a method for quantitative analyte identification in a sample, the method comprising the following steps being carried out by means of the point-of-care detection system according to any one of the embodiments of the second inventive aspect:*
*a) providing a sample in the plasmonic sensor cartridge according to any one of the embodiments of the first inventive aspect, wherein the sample is introduced within the sample reservoir;*
*b) routing, via the microfluidic cuvette, the sample from the sample reservoir towards the at least one functionalized region of the plasmonic sensor;*
*c) illuminating the at least one functionalized region of the plasmonic sensor with light from the illumination source;*
*d) polarizing the light in TM mode and directing the polarized light onto the metal surface of the plasmonic sensor at an oblique angle of incidence;*
*e) measuring, using the detection module, the intensity of light reflected from the at least one functionalized region; and*
*f) converting, via the processing unit, the reflected light intensity measured in step e) into a voltage signal; and*
*g) providing, by using the processing unit, a quantitative output related to the concentration of the analyte in the sample based on the voltage signal of step f).*

In this way, the method is based on correlating the measured reflected light intensity to a quantitative concentration of an analyte in the sample.

In an embodiment of the method, the illumination light is provided to the functionalized regions of the plasmonic sensor's surface as a uniform multisport illumination pattern.

In an embodiment of the method, the multi-spot illumination pattern is generated using a diffractive optical element.

In an embodiment of the method, step c) results in a plurality of sensing spots, and wherein each of said sensing spots is incident on the at least one functionalized region of the plasmonic sensor's surface.

In an embodiment of the method, the diffractive optical element is configured to generate a multispot illumination pattern such that a plurality of distinct sensing spots are defined on the at least one functionalized region.

In an embodiment of the method, the intensity of light reflected from each of the plurality of sensing spots is individually measured by a corresponding detection element of the detection module.

In an embodiment of the method, the at least one functionalized region comprises a plurality of spatially distinct functionalized sub-regions, and the at least one sensing spot is incident on each of said functionalized sub-regions for the simultaneous detection of multiple analytes.

In an embodiment of the method, step c) further comprises fine-adjusting the angular orientation of the plasmonic sensor cartridge relative to the incident illumination light of the illumination source by using a rotation stage to optimize the excitation of surface plasmon resonance prior to step e) of measuring the intensity of reflected light. After this fine-adjusting process, an optimal measurement angle is determined. This is a crucial step for maximizing the sensitivity of the measurement.

In an embodiment, multiple measurements are taken at the optimal measurement angle and voltage signal variation over time is analyzed. Advantageously, this embodiment allows monitoring the evolution of an analyte concentration in the sample.

The measurement method of the invention is based on real-time monitoring of changes in the reflected intensity at different functionalized regions of the plasmonic sensor's surface (i.e. after applying different sensing spots in functionalized regions), measured at a fixed angle. The operating principle relies on the fact that a variation in the resonance angle, due to a change in the refractive index, results in a corresponding change in the reflected intensity at a fixed incidence angle near the linear region of the plasmonic resonance peak. The evolution of these changes can be reproduced and visualized as quantitative linear sensorgrams.

In an embodiment, the method comprises at least the following parameters:
- Angular resolution (intensity vs. incidence angle) of the light reflected by the optical system within the angular range defined through surface plasmon resonance.
- Detection, adjustment, and interrogation of the resonance peak and its shift in incidence angle.
- Real-time visualization of variations in the reflected intensity at a fixed incidence angle near the linear region of the plasmonic resonance peak (intensity vs. time).

All features described in this specification, including the claims, description and drawings, can be combined in any way except for combinations of mutually exclusive features.

### General advantages of the cartridge and system of the invention over the prior art

Plasmonic sensing allows such rapid, sensitive and label-free testing but existing implementations suffer from poor multiplexed ability, complicated light coupling mechanisms and restrictive size implementation with bulky optics. Specific advantages of the claimed invention are listed below:
1. Multiplexed measurement: One single and minimal sample can be employed to detect multiple analytes of interest or disease biomarkers and track their binding in real-time.
2. Easy optical coupling: The invention does not require an optical prism and can be employed via a direct optical coupling with a myriad of different nanoplasmonics structures, simplifying the coupling requirements.
3. Economical: All the components are readily available, and the consumables are easy to produce at low and high volumes.
4. Compact: The simplified light coupling systems allows for a low-profile measurement device.
5. High sensitivity: The new sensor can match or exceed commercially available plasmonic platforms with sensitivities on the order of 10⁻⁵ refractive index units (RIU).

### Applications and use cases for the cartridge and the system according to the invention

The invention supports a diverse set of applications in many fields, including:
- Rapid infectious disease diagnosis: Enables quick, on-site testing for multiple pathogens, ideal for low-resource and high-burden settings.
- Biomarker screening: Facilitates early detection and ongoing monitoring of biomarkers related to disease state such as inflammatory markers, immune system activation markers, and cancer biomarkers.
- Food safety testing: Allows fast, multiplexed detection of pathogens and contaminants in food to prevent outbreaks.
- Environmental water monitoring: Provides real-time testing of pollutants in water sources to ensure safety and detect contamination early.
- Drug monitoring: Enables short-time analyses for continuous or semicontinuous monitoring of blood levels of drug with a narrow therapeutic range.
- Drug development screening: Supports high-throughput analysis of drug-target interactions, speeding up drug discovery and personalized medicine.

### DESCRIPTION OF THE DRAWINGS

To complement this description and with the aim of helping a better understanding of the characteristics of the invention, in accordance with specific embodiments, the following figures are attached as an integral part of said description where, for illustrative purposes and not limiting, the following has been represented:
- Figure 1: This figure shows a diagram with main elements of a point-of-care detection system embodiment according to the present invention.
- Figure 2: Detail of multispot illumination of the plasmonic sensor.
- Figure 3: Microfluidic cuvette embodiments designed to transfer the sample to the biofunctionalized regions on the surface of the plasmonic biosensor.
- Figure 4: Angular resolved measurements showing the behaviour of the plasmonic sensor.
- Figure 5: Characterizing the limit of detection (LOD) of the plasmonic sensor in terms of refractive index units (RIU) for three of the illumination regions and photodiode combinations, showing similar sensitivities across the plasmonic sensor surface.
- Figure 6: Sensorgrams showing the simultaneous output from three regions on the plasmonic sensor's surface when used in a biosensor application to detect the inflammatory biomarker C-reactive protein.
- Figure 7: Sensorgram showing the biosensor response in detecting the inflammatory biomarker C-reactive Protein (CRP) at three low concentrations.
- Figure 8: An embodiment of the microfluidic distribution unit for the invention.

### DETAILED DESCRIPTION OF THE INVENTION

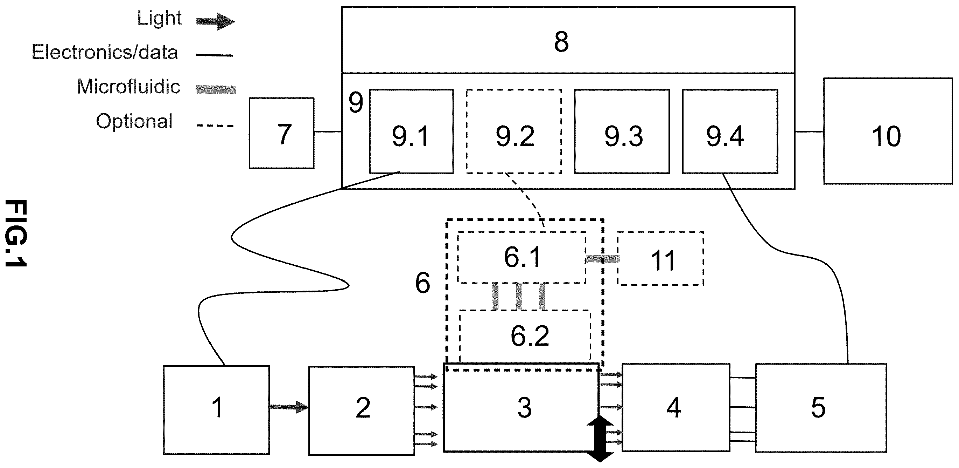

Figure 1 shows a block diagram of an embodiment of the point-of-care detection system according to the present invention. In this embodiment, the point-of-care detection system comprises a monochromatic illumination source (1) providing illumination, an optical arrangement (2) optically coupled to the illumination source (1), a plasmonic sensor cartridge (3), which we will refer to from now on as merely cartridge, optically coupled to the optical arrangement (2), a detection module (4) optically coupled to the plasmonic sensor cartridge (3) and an electronic unit (5) comprising detection electronics and control hardware.

As to the monochromatic illumination source (1), in the embodiment exemplifying the invention it is a laser diode.

As to the detection module (4), in the embodiment exemplifying the invention it is embodied as a photodiode array comprising a plurality of detection elements (4.1).

As explained more detail later, the invention conceives that the monochromatic illumination source (1), the optical arrangement (2), the detection module (4) and the electronic unit (5) be housed or commonly supported in a measurement device, the measurement device adapted to be able to insert and extract a cartridge (3), the cartridge being conveniently optically coupled between the optically arrangement (2) and the detection module (4) when inserted in the measurement device.

Figure 1 also shows optional microfluidic elements of the point-of-care detection system, particularly, an optional active microfluidic distribution unit (6).

This microfluidic distribution unit (6) comprises microfluidic pumps (6.1) and a microfluidic interface (6.2). The microfluidic interface (6.2) establishes a fluid communication between a plasmonic sensor (3.1) of the cartridge (3) and the microfluidic distribution unit (6). Also, the microfluidic distribution unit (6) may be in fluid communication with a waste reservoir (11). Although in this embodiment the microfluidic distribution unit (6) is active (i.e. it includes a pump), passive alternatives for this microfluidic distribution unit (6) are also possible.

From the electronics perspective, the embodiment of Figure 1 further comprises data storage means (7), a touchscreen (8), and a processing unit (9) which in turn comprises: a illumination source controller (9.1) configured to allow controlled operation of the illumination source (1), an optional microfluidic controller (9.2) (which is only required when an active microfluidic distribution unit (6) is included in the system), a Graphical User Interface, GUI (9.3), and a control and analysis software (9.4) including detection algorithms. Also, the system comprises a wireless communication module (10) configured to perform a data transfer with external devices.

In an embodiment of the system, the above referred touchscreen (8) interface as well as various connectivity features to allow data transfer off the system (such as via ethernet, Bluetooth, Wi-Fi) to secure data storage means (7) can be embodied in the above referred measurement unit. An embodiment of the system without a display and being controlled remotely from a personal computer or mobile device is, however, also possible. The system may naturally contain electronics and processing systems to control the light source (1), the electronic unit (5), to perform data acquisition, data processing, graphical user interface (9.3) and results display.

The cartridge (3) comprises a plasmonic sensor (3.1), which is the core sensing component of the point-of-care detection system.

The plasmonic sensor (3.1) is indeed contained within a consumable cartridge (3) which can be replaced for each sample test. Cartridges (3) may be tailored to detect a specific set of analytes. Anyway, each cartridge (3) comprises: a sample reservoir for the biological sample; the plasmonic sensor (3.1) which is functionalized to detect a specific set of analytes in regions on said plasmonic sensor (3.1); and a microfluidic cuvette adapted to distribute the sample to the active regions of the plasmonic sensor (3.1). The cartridge (3) may also include a casing for easy handling and may also include elements for identification of the sample, labelling and other features.

The plasmonic sensor (3.1) (also referred to as plasmonic chip) comprises in turn: a nanostructured metal surface, preferably a thin layer of gold (100 nm), deposited on a nanopatterned substrate. The said nanopatterned substrate can be fabricated either with custom nanoimprinting or, by repurposing commercial Blu-ray discs, which naturally contain a nanostructured pattern compatible with plasmonics excitation. The grating structure of this substrate allows coupling of the illumination onto the active region of the plasmonic sensor (3.1), interaction of plasmons with the active functionalized plasmonic sensor regions, and out-coupling of plasmons to light towards the detection elements (4.1) (i.e. photodetectors) of the detection module (4). Advantageously, by using a grating structure, light can be easily coupled to the cartridge (3) without need for prisms (as in most conventional surface plasmon resonance units) or complicated coupling optics.

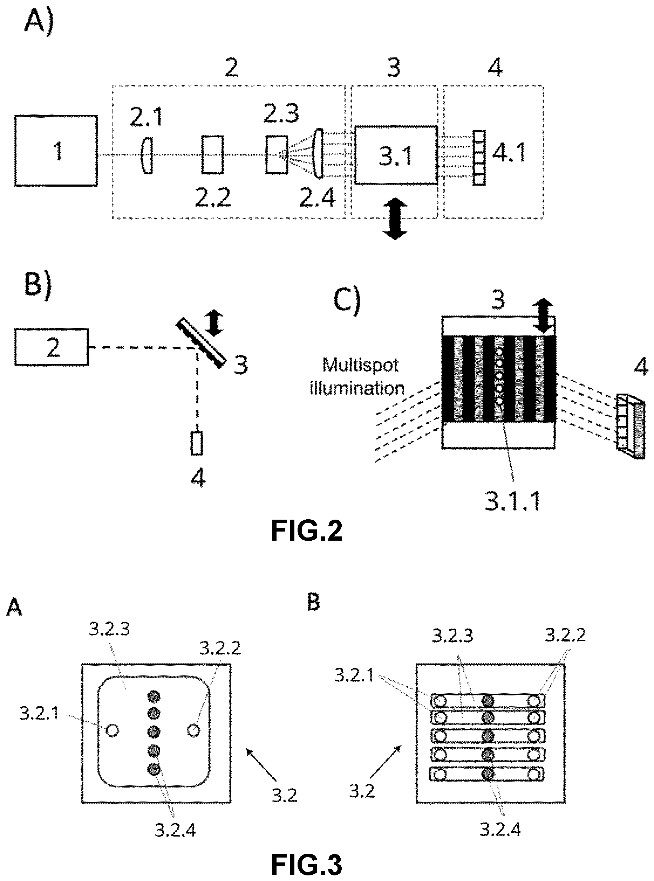

Figures 2A-C) provide further detail regarding the optical coupling between different elements of an embodiment of the point-of-care detection system.

Figure 2A) illustrates the optical arrangement (2), which comprises a collimator (2.1) optically connected to the illumination source (1), the collimator (2.1) optically interfacing with a polarizer (2.2), the polarizer (2.2) optically coupled with a diffractive optical element (2.3) (hereinafter referred to as 'DOE'), and the DOE (2.3) optically connected to an additional lens (2.4).

The polarizer (2.2) is configured to polarize the incident light in transverse magnetic (TM) polarization. The TM-polarized illumination exiting the polarizer (2.2) then impinges upon the DOE (2.3). The DOE (2.3) is configured to generate uniform multispot illumination and to direct this illumination towards the functionalized regions (3.1.1) of the plasmonic sensor (3.1). The additional lens (2.4) is configured to correct beam divergence and maintain the spatial separation between the output multispot illumination beams (labelled as "Multispot illumination" in Figure 2C)) as they propagate towards the plasmonic sensor (3.1). Thus, the light path originates from the illumination source (1), traverses the optical arrangement (2), and reaches the plasmonic sensor (3.1) within the cartridge (3). Finally, light reflected from the plasmonic sensor (3.1) is collected by the detection elements (4.1) of the detection module (4), which comprise a photodetector array.

As illustrated in Figure 2A), the light emitted by the illumination source (1) is collimated, polarized, and directed towards the plasmonic sensor (3.1). A DOE (2.3) then splits the incoming beam into multiple equispaced beams, creating multiple points of illumination on the plasmonic sensor's surface, enabling simultaneous measurements across several active regions. The DOE (2.3) is commercially available and can be selected based on the number of illumination points desired. Also, the polarizer (2.2), an optical filter and a lens (2.4) may be used to clean the laser beam, optimize the plasmonic excitation and create a uniform multispot pattern on the plasmonic sensor's surface.

Figure 2B) provides a top-down view of the system depicted in Figure 2A), illustrating the light path in this reflection-based configuration; and Figure 2C is a quasi-3D side view illustrating the light path from the optical arrangement (2) to the final detection on the detection elements (4.1) of the detection module (4), comprising an array of photodetectors, after interacting with the functionalized or active regions (3.1.1) of the plasmonic sensor (3.1).

In short, the point-of-care detection system comprises a measurement device for performing the evaluation, and plasmonic sensor cartridges (3) (including a casing, an embedded microfluidic distribution unit and the plasmonic sensor (3.1)). The plasmonic sensor (3.1) has various regions which are previously biofunctionalized to detect different analytes in the same sample of interest (e.g. human or biological sample). A drop of biological fluid (e.g. blood, plasma, urine) is placed on the sample reservoir of the cartridge (3) and, then said cartridge (3) is inserted into the measurement device. The sample fluid is either passively or actively driven by a microfluidic distribution unit (6) to several detection regions of the plasmonic sensor (3.1).

In the optical arrangement (2), a laser beam is divided into multiple beams by a holographic diffractive optical element DOE (2.3), such that to obtain a multisport illumination. Then, optics is used to direct these beams (i.e. the multispot illumination) to excite the multiple active/functionalized regions on the plasmonic sensor (3.1) which can detect different analytes present in the same sample. The detection optics and electronics monitors the plasmon resonance condition and detects changes in the local refractive index of the biosensor regions which corresponds to the binding of analytes to the biofunctionalized sensor surface. This binding can be monitored in real-time by the point-of-care detection system which is calibrated to allow quantitative concentration measurements of the analyte in the sample.

The cartridge (3) of point-of-care detection system comprises a microfluidic cuvette (3.2) that comprises one or more microfluidic channels to guide a sample from the sample reservoir towards the plasmonic functionalized regions (3.1.1) of the plasmonic sensor (3.1).

In different configurations, the microfluidic cuvette (3.2) can be designed for a single or multiple samples.

Figure 3 shows two microfluidic cuvette (3.2) designs to transfer the sample to the biofunctionalized regions on the surface of the plasmonic sensor (3.1). Illumination regions with biofunctionalized areas are displayed. One notices that the sensing spots provided by the collimated and polarized illumination after being splitted by the DOE (2.3) overlaps the functionalized regions (3.1.1) of the plasmonic sensor (3.1). For instance, the sample may flow in an active microfluidic configuration being pumped by the microfluidic pump (6.1). As illustrated in Figure 3, the plasmonic sensor can receive single or multiple sample inputs, directing them towards one or more distinct active regions of the sensor's surface.

Figure 3A) corresponds to a microfluidic cuvette (3.2) embodiment having a single microfluidic sample channel (3.2.3) on the plasmonic sensor cartridge (3). This design comprises a microfluidic input (3.2.1), a microfluidic output (3.2.2) and a microfluidic sample channel (3.2.3) to transfer a sample from the microfluidic input (3.2.1) to the microfluidic output (3.2.2). Biofunctionalized sensing regions (3.2.4) of the plasmonic sensor's surface are also shown, which overlap illumination regions. In this case, the microfluidic cuvette (3.2) is designed with a single sample channel (3.2.3) that covers the functionalized region (3.1.1) of the plasmonic sensor's (3.1) surface with M=5 detection spots. In other embodiments, a different M value is possible. The material of the cuvette (3.2) is important (as in any biosensor) for biocompatibility, and the choice of this material depends on aspects such as transparency and the quality of the sensing window since the biosensor device operates in reflection, and therefore with direct incidence of light on the surface of the plasmonic sensor and through the microfluidic cuvette (3.2).

In the embodiment of Figure 3A), the optical arrangement (2) is configured to provide M light spots, where M is an integer value larger than 3, preferably M is between 3 and 11. M light spots are distributed in the functionalized region (3.1.1), to cover it. Advantageously, by having M distinct detection spots within the same flow channel and interacting with the same functionalized region after a single introduction of the sample, the same measurement is performed M times in parallel or in close succession as the sample flows across these spots. Thus, the configuration of Figure 3A) can be useful to repeat the same measurement M times, thus minimizing the error of the measurement through averaging.

Figure 3B) shows an alternative embodiment of the microfluidic cuvette (3.2) comprising multiple microfluidic sample channels (3.2.3) on a same cartridge (3). In that case, multiple individual sample channels (3.2.3) exist putting in fluid communication a plurality of microfluidic inputs (3.2.1) and corresponding microfluidic outputs (3.2.2). By providing dedicated sample channels (3.2.3), multiple analytes can be assessed simultaneously. Also, each individual sample channel (3.2.3) can be designed to direct the sample over a specific functionalized region (3.1.1) tailored to bind a particular target analyte. Advantageously, the physical separation provided by the independent sample channels (3.2.3) minimizes the risk of cross-contamination between different analytes or their respective sensing regions.

In an embodiment, the microfluidic cuvette (3.2) is made from a variety of biocompatible, bio-resistant low-cost polymers (e.g. PMMA, PDMS) or other suitable materials and is hermetically secured on top of the plasmonic biosensor (3.1).

In some embodiments, the microfluidic cuvette (3.2) has an active configuration. In those embodiments, the microfluidic cuvette (3.2) is in fluid communication with microfluidic pumps (6.1), waste reservoirs (11) and valves in the measurement device through input and output ports on the cartridge (3), to control the flow of samples to the active sensing regions of the plasmonic sensor's surface.

In alternative embodiments, the microfluidic cuvette (3.2) is passively driven. In those embodiments, the sample is applied to at least an input port of the cartridge (3) and is redistributed passively, e.g. through capillary action, towards the active sensing part of the plasmonic sensor (3.1).

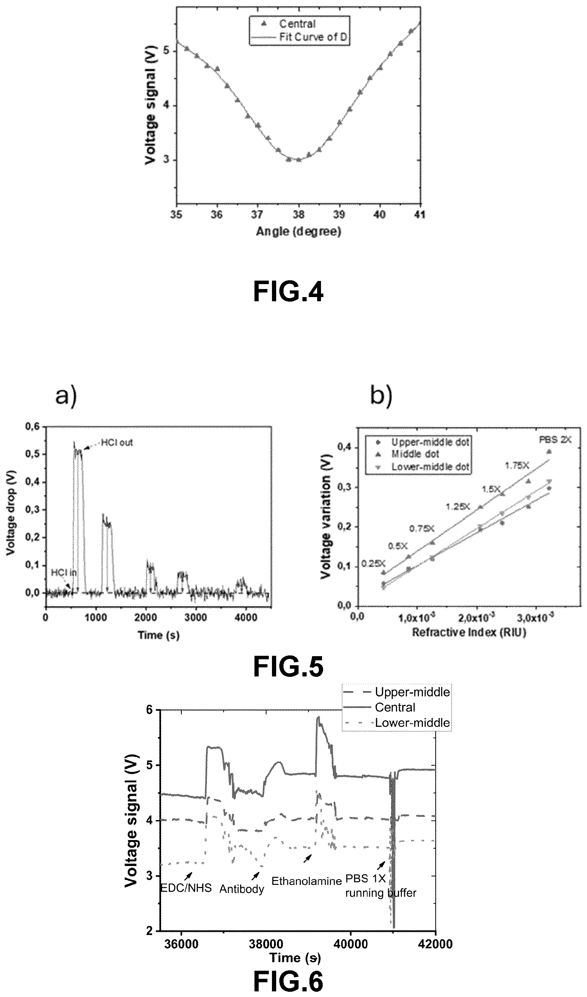

Figure 4 shows angular resolved measurements showing the behaviour of the plasmonic sensor (3.1) of a point-of-care detection system according to the invention. Each point corresponds to a change of the light incidence angle with the rotating of a rotating stage with respect to the incidence light (in steps of 0.2-0.3 degrees) to cover the 6-degree range that defines the resonance peak. The collected light in the detection module (4) (photodiode array) changes (decreases) as the optimal angle where the resonance takes place approaches (the angle at the minimum voltage), located at 38 degrees for the tested plasmonic sensor (3.1).

In that embodiment, the plasmonic sensor (3.1) comprises a nanopatterned substrate with pit depth of 25 nm, and a metal surface coating the nanopatterned substrate having a thickness of 100 nm thickness. In this embodiment, the metal surface coating is made of gold and the periodicity between pits of the substrate is 320 nm. The illumination source (1) used to carry out these measurements has been a monochromatic, polarized (TM polarized) laser diode emitting at a wavelength around 660 nm. This wavelength is chosen to match the plasmonic resonance for the nanograting structure. The specific plasmonic sensor (3.1) applied in the experimental assays of the present disclosure comprises:
- a nanopatterned substrate comprising a plurality of pits separated by lands; wherein the pit depth is between 25 nm and the separation between adjacent pits is between 320 nm, wherein the width of the pits is 160 nm;
- a gold metal surface coating the nanopatterned substrate and having a thickness of 100 nm.

For these tests, no nanostructured adhesion layer is employed between the substrate and the surface coating. To obtain this plasmonic sensor (3.1), a commercial Blu-ray disc has been adapted according to the following manufacturing process:
- the Blu-ray disc is provided; this disc which comprises a nanostructured polycarbonate substrate;
- the upper reflective and protective layer of the Blu-ray disc is removed;
- a metal surface coating of gold is directly vaporized over the nanostructured polycarbonate substrate of the Blu-ray disc.

The same system (including the same cartridge) is used for the tests included in the following figures.

Figure 5(a) presents real-time sensorgrams illustrating the temporal evolution of the voltage signal acquired by the detection module (4) (photodetector array) at a fixed detection angle. These sensorgrams correspond to the injection of hydrochloric acid (HCl) solutions of varying concentrations, and thus different refractive indices, through the microfluidic system to the sensing region. The sensorgrams demonstrate an increase in baseline voltage upon an increase in the refractive index of the introduced solution and a subsequent decrease as the solution is flushed from the sensing regions of the plasmonic sensor's surface.

Figure 5(b) illustrates the characterization of the sensitivity and limit of detection (LOD), expressed in refractive index units (RIU), for three distinct illumination regions of a system embodiment, revealing comparable values across the system. This indicates that the sensing spot position on the plasmonic sensor's surface does not significantly impact the system's sensitivity. Each calibration curve, derived through linear regression, represents the voltage variations as a function of changes in refractive index, achieved using a series of phosphate-buffered saline (PBS) solutions with varying salt concentrations. The sensitivity is determined from the slope of the linear regression, and the LOD for each illumination configuration (corresponding to each sensing spot position) is calculated based on the noise level of the plasmonic biosensor and the determined sensitivity. A summary of the sensitivity values achieved by this system embodiment is presented in the table below.

| **Sensing spot position** | Upper-middle | Middle | Lower-middle |
|---|---|---|---|
| **LOD (10⁻⁵ RIU)** | 1.02 | 0.81 | 0.49 |

Figures 6-7 show an example case of the binding of low concentration of C-reactive protein (an inflammatory biomarker) detected by using an embodiment of the system according to the invention.

Figure 6 presents real-time sensorgrams illustrating the temporal evolution of the voltage signal obtained from three distinct regions of the plasmonic biosensor (labeled as 'Upper-middle,' 'Central,' and 'Lower-middle') during biosensing measurements for the detection of a protein biomarker. The sensorgrams in Figure 6 depict the process of surface activation and biofunctionalization of the biosensor plasmonic chip with antibodies through a three-step reaction: a) injection of an activation solution comprising 0.2 M 1-Ethyl-3-(3-dimethylaminopropyl)carbodiimide (EDC) and 0.05 M N-hydroxysuccinimide (NHS) dissolved in an aqueous buffer (100 mM MES at pH 5.5); b) injection of an antiC-reactive protein (anti-CRP C7) antibody solution at a concentration of 20 µg/mL in 100 mM MES at pH 5.5; and c) injection of a blocking solution consisting of 1 M ethanolamine at pH 8 for 2 minutes. Milli-Q water was used as the running buffer. In this case, a constant flow rate of 20 µL/min was maintained throughout the experiment. The observed changes in the voltage signal in response to the injection of different solutions demonstrate the sensitivity of the point-of-care detection system to changes in the plasmonic sensor's surface environment.

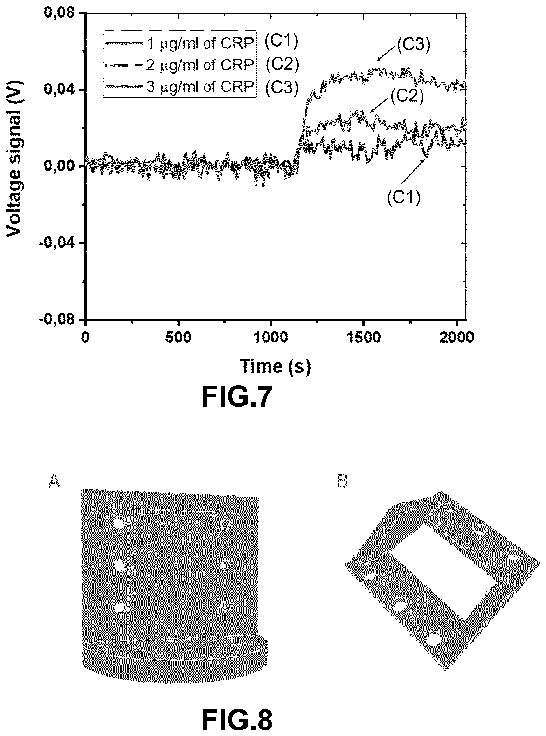

Figure 7 illustrates real-time sensorgrams showing the plasmonics (bio)sensor response (evolution of the voltage) after injection of three different concentrations (C1, C2 and C3) of CRP protein over an anti-CRP biofunctionalized sensor chip. The specific biointeraction between the antibody and its target protein is visualized in real-time as a capture event on the surface, which increases the local refractive index (i.e. an increase in the voltage signal). The voltage signal is proportional to the concentration of the target analyte as the number of the biointeraction events increases.

The previous experimental tests demonstrate that the point-of-care detection system according to the present invention enables simultaneous detection of multiple biomolecular interactions from a single (or multiple) sample on a plasmonic sensor cartridge (3). The system uses plasmonic nanostructures for direct light coupling (such as grating-based Blu-ray discs as the substrate), thereby eliminating the need for prisms and reducing size and cost. In preferred embodiments of the system, a DOE (2.3) generates multiple illumination spots, achieving multiplexing capabilities without requiring mechanical moving parts. The point-of-care detection system of the invention integrates a microfluidic distribution unit for automated handling of small sample volumes and achieves high sensitivity (in the order of ~10⁻⁵ RIUs), comparable to other plasmonic sensor systems.

Figure 8 shows an embodiment of the microfluidic distribution unit (6) for the invention. It consists of a support made by 3D printing with standard DLP (Digital Light Processing) resin that anchors the plasmonic chip in the frame (cf. Figure 8A). The microfluidic is defined by a single channel (e.g. 75 µm high), defined by double-sided adhesive tape of this thickness. This is a commercial adhesive tape marketed specifically for this purpose and is sealed at the top with a transparent window based on a flat cover made of COC polymer with two holes for connecting the microfluidic tubes. The complete cartridge incorporates an extra element (illustrated in figure 8B) also manufactured by 3D printing to ensure complete anchorage.

An operational workflow example using an embodiment of the system according to the present invention comprises the following steps:
- Sample preparation and plasmonic sensor cartridge (3) introduction in the measurement device of the invention system: The sample to be analyzed (e.g., blood, serum, or other aqueous solution) is introduced onto a sample reservoir of the cartridge (3). The cartridge (3) is then inserted into the measurement unit. The sample is then redistributed actively or passively to the active sensing regions (i.e. functionalized regions) of the plasmonic sensor (3.1), to sense different analytes. In another embodiment, multiple biological samples can be placed on the plasmonic sensor cartridge (3), and microfluidics individually routes each sample to a specific plasmonic sensor (3.1) region.
- Illumination and plasmonics excitation: The polarized illumination source (1) (like a laser diode) directs light towards the plasmonics sensor (3.1), where the DOE (2.3) splits it into multiple beams. These beams are projected onto specific spots on the plasmonic sensor surface, thereby creating a multispot illumination pattern. The spots align to different plasmonic sensing regions (i.e. active regions of the plasmonic sensor's surface), which can have different biofunctionalization to detect different analytes in the same sample.
- Multiplexed sensing: As the sample flows over the plasmonics sensor (3.1), the nanostructured surface induces a plasmonic resonance, resulting in a resonant reflection at specific angles. Any binding events on the plasmonic sensor's surface, such as antigen-antibody interactions, cause local refractive index changes, shifting the resonance angle and altering the reflected light intensity.
- Real-time detection and data acquisition: The photodetector array captures the reflected light from each illuminated spot and monitors intensity changes in real-time. A system's software may automatically optimize integration time and performs polynomial fitting to track intensity shifts, generating quantitative sensorgrams for each spot.
- Signal processing and analysis: After a calibration, the data acquisition software calculates analyte concentration based on the changes in light intensity on the sensor. The system displays real-time data, including intensity vs. time curves, allowing users to monitor binding events and analyze results instantly. The calibration of a sensor for the detection of a given analyte is performed, in its classical form, by constructing a calibration curve. To do this, different samples of known concentration are measured, representing the values obtained in a graph where the X-axis corresponds to the concentration and the Y-axis to the signal recorded by the sensor. With this curve, when measuring samples of unknown concentration, the corresponding signal is determined and interpolated on the curve to estimate the concentration. However, from a commercial point of view, with an already fully developed and optimised illumination beam and system, calibration can be simplified. In these cases, a standard of known concentration is periodically injected or measured. The signal obtained from this standard is used to adjust or correct subsequent measurements in real time, allowing the accuracy of the sensor to be maintained without the need to repeat the entire extensive calibration process.
- Post-measurement: The plasmonic sensor cartridge (3) is removed from the system and disposed or sent for recycling.

Biosensor measurements using the system of the present invention, specifically the detection of a target analyte or marker, can be achieved in under 10 minutes. In a laboratory setting, an optional regeneration step may be included to desorb the bound analyte, thereby restoring the plasmonic sensor surface with the bioreceptor (e.g., antibody) for subsequent measurements. When both detection and regeneration steps are performed, the total measurement cycle time can range from 10 to 25 minutes. This methodology allows for continuous measurements, as the plasmonic sensor can be regenerated multiple times up to its experimentally determined active lifetime (e.g., limited by the degradation of the biological material on its surface). Consequently, the plasmonic sensor cartridge can be reused.

For the measurement, the intensity of the incident light that has been reflected from the plasmonic chip is collected with the photodetector, and the photodetector converts it into an electrical signal, the output being a voltage. As long as the light is incident, the signal will be collected, continuously. At the operational and practical level, the system can be acquiring data continuously, assuming that samples are injected successively. At the end device level, it would be operational every time an analysis is performed, which can take between 10-25 min.

In other suitable embodiments, particularly for commercial systems, the plasmonic sensor cartridges (3) are intended for single use. In such cases, once a plasmonic sensor (3.1) is placed within the cartridge (3), a measurement can be obtained within a few minutes (5-10 minutes).

## Claims

1. A plasmonic sensor cartridge (3) for a point-of-care detection system for quantitative analyte identification in a sample to be analyzed, the cartridge (3) comprising:
• a sample reservoir adapted to contain the sample to be analyzed;
• a plasmonic sensor (3.1) configured to detect the concentration of a specific analyte of the sample, the plasmonic sensor comprising at least one functionalized region (3.1.1);
• a microfluidic cuvette (3.2) in fluid communication with the plasmonic sensor (3.1) and configured to distribute the sample from the sample reservoir towards the at least one functionalized region (3.1.1);
wherein the plasmonic sensor (3.1) comprises:
• a nanopatterned substrate comprising a plurality of pits separated by lands; wherein the pit depth is in the range of 25 to 50 nm and the separation between adjacent pits is in the range of 200 to 750 nm;
• a metal surface coating the nanopatterned substrate and having a thickness between 50-150 nm, the metal surface being configured to support surface plasmon resonance, wherein the metal surface is functionalized for the selective binding of the analyte.

2. The plasmonic sensor cartridge (3) of the preceding claim, further comprising a casing integrating the sample reservoir, the microfluidic cuvette and the plasmonic sensor.

3. The plasmonic sensor cartridge (3) of any one of claims 1 to 2, wherein the metal surface is made of a plasmonic metal, the plasmonic metal preferably comprising gold, silver or aluminium; and wherein the metal surface has a thickness between 50 and 150 nm.

4. The plasmonic sensor cartridge (3) of any one of claims 1 to 3, wherein the nanopatterned substrate comprises one of the following: a Blu-ray disc, polymeric nanopatterned substrate, a metallic nanograting, a silica nanograting.

5. The plasmonic sensor cartridge (3) of any one of claims 1 to 4, wherein the microfluidic cuvette comprises: a plurality of microfluidic channels to route the sample from the reservoir towards the at least one functionalized region (3.1.1), and at least a waste reservoir.

6. The plasmonic sensor cartridge (3) of any one of claims 1 to 5, which is manufactured by a biocompatible material selected from the group comprising one or more of the following: PDMS, SU-8, PMMA, COC/COP, PC, PFTE, PFA, FEP.

7. The plasmonic sensor cartridge (3) of any one of claims 1 to 6, wherein the plasmonic sensor (3.1) comprises a plurality of functionalized regions (3.1.1) functionalized for the selective binding of different analytes in the sample.

8. The plasmonic sensor cartridge (3) of any one of claims 1 to 7, wherein:
• the cartridge is sealed to prevent leakage of the sample; and/or
• the volume of the sample reservoir is between 50-150 µL.

9. A point-of-care detection system for quantitative analyte identification in a sample, the system comprising:
a) the plasmonic sensor cartridge (3) according to any one of claims 1 to 8;
b) a measurement device comprising one or more of the following:
• a microfluidic distribution unit (6) configured to route the sample to the plasmonic sensor cartridge (3); wherein the microfluidic cuvette (3.2) of the cartridge (3) is in fluid communication with said microfluidic distribution unit (6);
• a monochromatic illumination source (1) configured to emit light within the visible to near infrared spectrum range and to couple light into the at least one functionalized region (3.1.1) of the plasmonic sensor (3.1);
• optical arrangement (2) configured to polarize the light from the illumination source (1) in TM mode and to direct the polarized light onto at least onto the metal surface of the plasmonic sensor (3.1) at an oblique angle of incidence;
• a detection module (4) optically coupled with the plasmonic sensor cartridge (3) and configured to measure the intensity of the light reflected from at least one functionalized region of the metal surface of the plasmonic sensor (3.1) after interaction of the polarized light with said plasmonic sensor (3.1);
c) a processing unit (9) communicably coupled to the measurement device and configured to:
• convert the reflected light intensity into a voltage signal; and
• provide a quantitative output representative of the concentration of the analyte based on said voltage signal.

10. The system of the preceding claim, wherein the monochromatic illumination source (1) is a laser diode configured to emit at a wavelength in the visible and/or near-infrared range around 660 nm.

11. The system of any one of claims 9 to 10, wherein the optical arrangement (2) further comprises:
• a collimator (2.1) optically coupled to the monochromatic illumination source (1) and configured to collimate a light beam emitted by the monochromatic illumination source (1);
• a polarizer (2.2) optically coupled to the collimator and configured to generate a transverse magnetically polarized collimated light beam;
• a diffractive optical element (2.3) optically coupled to the polarizer and configured to shape the polarized light beam and direct said polarized light beam towards the plasmonic sensor (3.1) of the plasmonic sensor cartridge (3); wherein said diffractive optical element (2.3) is further configured to generate a multispot illumination pattern incident on the at least one functionalized region (3.1.1), thereby defining a plurality of sensing spots onto the at least one functionalized region (3.1.1).

12. The system of any one of claims 10 to 11, wherein the detection module (4) comprises:
• a plurality of detection elements (4.1) optically coupled with the plasmonic sensor (3.1), said detection elements (4.1) being selected from the group comprising a photodiode array, a CMOS camera or a CCD camera configured to detect light in the visible to near infrared spectrum range;
• wherein the plurality of detection elements (4.1) is configured to measure the intensity of the light reflected by the at least one functionalized region (3.1.1).

13. The system of the preceding claim, wherein the detection elements (4.1) are provided as a photodiode array comprising up to N=16 channels, each channel being associated with a different sensing spot of the multispot illumination incident on a corresponding functionalized region (3.1.1) of the plasmonic sensor (3.1).

14. The system of any one of claims 9 to 13, wherein the microfluidic distribution unit (6) is configured to deliver, in a controlled manner, the sample to the microfluidic cuvette (3.2); the microfluidic distribution unit (6) comprising:
• A pump (6.1) configured to deliver liquid solutions at a constant flow rate to the plasmonic sensor (3.1) via the microfluidic cuvette (3.2);
• A liquid tubing system arranged between the pump and the plasmonic sensor (3.1) and configured to provide fluid communication between the pump (6.1) and the plasmonic sensor (3.1);
• an optional injection valve in fluid communication with the liquid tubing system and configured to load and inject successive samples towards the plasmonic sensor (3.1); and
• a waste reservoir adapted to collect waste solutions.

15. A method for quantitative analyte identification in a sample, the method comprising the following steps being carried out by means of the point-of-care detection system according to any one of claims 9 to 14:
a) providing a sample in the plasmonic sensor cartridge (3) according to any one of claims 1 to 8, wherein the sample is introduced within the sample reservoir;
b) routing, via the microfluidic cuvette (3.2), the sample from the sample reservoir towards the at least one functionalized region (3.1.1) of the plasmonic sensor (3.1);
c) illuminating the at least one functionalized region (3.1.1) of the plasmonic sensor (3.1) with light from the illumination source (1);
d) polarizing the light in TM mode and directing the polarized light onto the metal surface of the plasmonic sensor (3.1) at an oblique angle of incidence;
e) measuring, using the detection module (4), the intensity of light reflected from the at least one functionalized region (3.1.1); and
f) converting, via the processing unit (9), the reflected light intensity measured in step e) into a voltage signal; and
g) providing, by using the processing unit (9), a quantitative output related to the concentration of the analyte in the sample based on the voltage signal of step f).
